# EUROPEAN PATENT SPECIFICATION

(11) **EP 3 215 135 B1**
(45) Date of publication and mention of the grant of the patent: **13.05.2020**
(21) Application number: 15857385.7
(22) Date of filing: 06.11.2015
(51) Int. Cl.: A61K 31/517, A61K 31/55, A61K 31/445, A61K 31/5517, A61K 31/551, A61K 31/506, A61K 31/519, A61P 35/00

(54) **METHODS TO TARGET TRANSCRIPTIONAL CONTROL AT SUPER-ENHANCER REGIONS**
VERFAHREN ZUR ZIELTRANSKRIPTIONSSTEUERUNG IN SUPER-ENHANCER-REGIONEN
MÉTHODES POUR CIBLER LE CONTRÔLE TRANSCRIPTIONNEL AU NIVEAU DES RÉGIONS SUPER-AMPLIFICATRICES

(30) Priority: 07.11.2014 US 201462077042 P; 20.04.2015 US 201562150110 P
(43) Date of publication of application: 13.09.2017
(73) Proprietor: Tolero Pharmaceuticals, Inc., Lehi, UT 84043 (US)
(72) Inventor: WARNER, Steven L., Sandy, Utah 84094 (US); BEARSS, David J., Alpine, Utah 84004 (US)
(74) Representative: Hoffmann Eitle
(86) International application number: PCT/US2015/059566
(87) International publication number: WO 2016/073913

(56) References cited:
- WO-A1-2016/061144
- WO-A2-03/028001
- WO-A2-2015/070020
- US-A1- 2014 303 167
- W. FISKUS ET AL: "Highly Active Combination of BRD4 Antagonist and Histone Deacetylase Inhibitor against Human Acute Myelogenous Leukemia Cells", MOLECULAR CANCER THERAPEUTICS, vol. 13, no. 5, 1 May 2014 (2014-05-01), pages 1142-1154, XP055235185, US ISSN: 1535-7163, DOI: 10.1158/1535-7163.MCT-13-0770
- Tomoaki Fukui ET AL: "The Analysis of the Effect of JQ1 and Flavopiridol on Chondrocytes under Inflammatory Stimuli", ORS 2014 Annual Meeting, 15 March 2014 (2014-03-15), XP055275562, Retrieved from the Internet: URL:http://www.ors.org/Transactions/60/125 1.pdf [retrieved on 2016-05-26]
- FISKUS ET AL.: 'Highly Active Combination of BRD4 Antagonist and Histone Deacetylase Inhibitor against Human Acute Myelogenous Leukemia Cells' SMALL MOLECULE THERAPEUTICS vol. 13, no. 5, 16 January 2014, pages 1142 - 1154, XP055235185
- FALKENBERG ET AL.: 'Histone deacetylases and their inhibitors in cancer, neurological diseases and immune disorders' NATURE REVIEWS DRUG DISCOVERY vol. 13, no. 9, 18 August 2014, pages 673 - 691, XP055440067
- FILIPPAKOPOULOS ET AL.: 'Targeting bromodomains: epigenetic readers of lysine acetylation' NATURE REVIEWS DRUG DISCOVERY vol. 13, no. 5, 22 April 2014, pages 337 - 356, XP055214318

## Description

### BACKGROUND

The invention is limited to the subject-matter defined in the appended claims; the following description is limited to this limitation.

### Technical Field

The present invention is generally directed to compositions for use in methods for treatment of diseases modulated by super-enhancers and compositions for the same.

### Description of the Related Art

Super-enhancers are large clusters of transcriptionally active regions of DNA that drive the expression of genes that control cell identity. Super-enhancers become dysregulated in multiple disease states, including but not limited to, cancer. Super-enhancers recruit transcription factors, cofactors, chromatin regulators, signaling enzymes (*e.g.,* kinases) and the transcriptional machinery (*e.g.,* RNA polymerase II) that form a large complex that regulates the expression of multiple genes simultaneously that are not necessarily in close proximity in regard to the linear form of DNA (Smith and Shilatifard, 2014, Nature Structural and Molecular Biology 21(3):210-219). Super-enhancers allow cells to have efficiencies in regulating groups of genes that work in concert to determine or maintain cell identity. It has been demonstrated that cancer cells reprogram super-enhancer complexes to change the transcriptional activity of a cancer cell leading to oncogenesis, metastasis, and progression of the disease. In fact, it has been postulated that many diseases, even outside of cancer, are ultimately a result of malfunctioning super-enhancer complexes (Cell 2013, Nov 7;155(4):934-47).

Known components of the super-enhancer complex include cyclin-dependent kinases (e.g., Cdk9), bromo and extra terminal (BET) domain proteins (e.g., Brd4), histone deacetylases (HDACs), histone acetyltransferases (e.g., p300), histone demethylases (e.g., Lsd1), histone methyltransferase (e.g., Dot1L), and others. These components are generally involved in typical transcriptional regulation. However, inhibiting components of super-enhancers function has been shown to have a more profound effect on super-enhancer controlled genes as compared to genes controlled by typical enhancer elements (Lovén et al., 2013,.Cell 153(2):320-334). One of the possible reasons for this observation is the enrichment of transcriptional and chromatin modifying proteins at super-enhancers as compared with typical enhancers. For instance, the Mediator complex (which includes Cdk8) and Brd4 are present at both super-enhancers and typical enhancers. However, the amount of Mediator at a super-enhancer compared with a typical enhancer has been found to be in excess of 25-fold (Hnisz, et al., 2013, Cell 155:934-947). Similarly, the level of Brd4 at super-enhancers has been shown to be approximately 20-fold higher than at typical enhancers (Hnisz, et al., 2013, Cell 155:934-947).

Genes expressed from super-enhancers have been shown to mediate disease progression in some cancers. For instance, in multiple myeloma (MM) tumor cells often have a translocation that places a super-enhancer element adjacent to the MYC gene (Hnisz, et al., 2013, Cell 155:934-947). Similar alterations have been found in patients with acute lymphoblastic leukemia (T-ALL), lung cancer, pancreatic cancer, colorectal cancer, breast cancer, chronic myelogenous leukemia (CML), glioblastoma, lymphoblastoid, cervical cancer and prostate cancer (Hnisz, et al., 2013, Cell 155:934-947).

While progress has been made, there remains a need in the art for improved targeting of super enhancers and methods for treating diseases mediated by super enhancers. The present invention fulfills this need and provides related advantages.

### BRIEF SUMMARY

In brief, the present disclosure relates to methods for treatment of disease involving administration of inhibitors of two of more components of super-enhancers. In one embodiment the disclosure provides a method for treating a super-enhancer-mediated disease in a mammal in need thereof, the method comprising administering to the mammal an effective amount of at least two of the following therapeutic agents:
i) a cyclin-dependent kinase inhibitor;
ii) a bromodomain inhibitor;
iii) a histone methyltransferase inhibitor;
iv) a histone deacetylase inhibitor; and
v) a histone demethylase inhibitor.

Pharmaceutical compositions comprising at least two of the foregoing therapeutic agents are also provided.

These and other aspects of the invention will be apparent upon reference to the following detailed description.

### BRIEF DESCRIPTION OF THE DRAWINGS

In the figures, identical reference numbers identify similar elements. The sizes and relative positions of elements in the figures are not necessarily drawn to scale and some of these elements are arbitrarily enlarged and positioned to improve figure legibility. Further, the particular shapes of the elements as drawn are not intended to convey any information regarding the actual shape of the particular elements, and have been solely selected for ease of recognition in the figures.
Fig. 1 illustrates a proposed mechanism for Brd4 recruitment of Cdk9 to transcriptional start sites.
Fig. 2 depicts evidence of synergistic effect between Cdk9 and Brd4 inhibitors.
Fig. 3 shows body weight gain for mice treated with vehicle, alvocidib, JQ1 or a combination of alvocidib and JQ1.
Fig. 4 provides evidence of synergistic effect between Cdk9 and Brd4 inhibitors at the level of super-enhancer mediated transcription.
Figs. 5A and 5B are bar graphs showing inhibition of MYC (Fig. 5A) and MCL-1 (Fig. 5B) expression by alvocidib in MV4-11 cells.
Fig. 6 presents apoptosis data for MV4-11 cells treated with alvocidib or BRD4 inhibitors alone and in combination.
Figs. 7A-C are graphs showing MYC expression in the presence of alvocidib, JQ1 (7A), OTX (7B) and IBET762 (7C) as single agents and in combination.
Figs. 8A-C are western blots for MYC expression in the presence of alvocidib, JQ1 (8A), OTX (8B) and IBET762 (8C) as single agents and in combination.
Figs. 9A-C are bar graphs showing quantification of the western blots of Figs. 8A-C. Data for JQ1 is provided in Fig. 8A, OTX015 data is provided in Fig. 8B and data for IBET762 is presented in Fig. 9C.
Fig. 10A is a representative western blot for MV4-11 cells treated with alvocidib. Fig. 10B provides quantification of the western blot of Fig. 10A.
Fig. 11A is a representative western blot for a dose escalation experiment in MV4-11 cells treated with alvocidib. Fig. 11B provides quantification of the western blot of Fig. 11A.
Fig. 12A is a representative western blot for MV4-11 cells treated with alvocidib for 2 hours at different doses. Fig. 12B provides quantification of the western blot of Fig. 12A.
Fig. 13A is a representative western blot for a dose escalation experiment in A549 cells treated with alvocidib. Fig 13B is a representative western blot for A549 cells treated with alvocidib for specific time periods.
Fig. 14A is a representative western blot for a time dependent experiment for A549 cells treated with alvocidib. Fig. 14B shows the same for SK-N-AS cells.
Fig. 15 provides quantification of mRNA repression in MV4-11 cells while treating with alvocidib in a time dependent manner.
Fig. 16A-C are graphs showing MCL-1 expression in the presence of alvocidib, JQ-1 (16A), OTX (16B) and IBET762 (16C) as single agents and in combination.
Fig. 17 is a graph showing MCL-1 expression in the presence of alvocidib and JQ-1 as single agents and in combination across a range of dosing concentrations.
Fig. 18 is a representative western blot showing c-MYC expression in A549 cells in the presence of alvocidib and JQ-1 as single agents and in combination
Fig. 19 presents apoptosis data for A549 cells in the presence of alvocidib or JQ-1 inhibitors alone and in combination.
Fig. 20 presents apoptosis data for A549 cells in the presence of dinaciclib or JQ-1 inhibitors alone and in combination.
Fig. 21A and Fig. 21B are graphs showing MYC and MCL-1 expression in A549 cells in the presence of alvocidib across a range of dosing concentrations.
Fig. 22A is a representative western blot showing CDK9 levels untreated, treated with non-targeting siRNA, and treated with CDK9-specific siRNA. Fig. 22B presents apoptosis data for A549 cells when treated with BET inhibitors and either non-targeted or CDK9-specific siRNA.

### DETAILED DESCRIPTION

In the following description, certain specific details are set forth in order to provide a thorough understanding of various embodiments of the invention. However, one skilled in the art will understand that the invention may be practiced without these details.

Unless the context requires otherwise, throughout the present specification and claims, the word "comprise" and variations thereof, such as, "comprises" and "comprising" are to be construed in an open, inclusive sense, that is, as "including, but not limited to".

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

"Mammal" includes humans and both domestic animals such as laboratory animals and household pets (e.g., cats, dogs, swine, cattle, sheep, goats, horses, rabbits), and non-domestic animals such as wildlife and the like.

A "pharmaceutical composition" refers to a formulation of a compound of the invention and a medium generally accepted in the art for the delivery of the biologically active compound to mammals, e.g., humans. Such a medium includes all pharmaceutically acceptable carriers, diluents or excipients therefor.

"Effective amount" or "therapeutically effective amount" refers to that amount of a compound of the invention which, when administered to a mammal, preferably a human, is sufficient to effect treatment, as defined below, of a super-enhancer related condition or disease in the mammal, preferably a human. The amount of a compound of the invention which constitutes a "therapeutically effective amount" will vary depending on the compound, the condition and its severity, the manner of administration, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or condition of interest, and includes:
(i) preventing the disease or condition from occurring in a mammal, in particular, when such mammal is predisposed to the condition but has not yet been diagnosed as having it;
(ii) inhibiting the disease or condition, i.e., arresting its development;
(iii) relieving the disease or condition, i.e., causing regression of the disease or condition; or
(iv) relieving the symptoms resulting from the disease or condition, i.e., relieving pain without addressing the underlying disease or condition. As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

### I. METHODS

In certain embodiments, the methods are useful for disrupting super-enhancer function and/or for preventing, treating, or ameliorating of a symptom associated with a disease, disorder, or pathological condition involving super-enhancer function, preferably one afflicting humans. Compounds which, when administered together or sequentially, inhibit the activity of two or more components of super-enhancers will be useful in preventing, treating, ameliorating, or reducing the symptoms or progression of cancer, such as acute myeloid leukemia (AML) and lung cancer. The present invention provides methods for inhibiting super-enhancers comprising administering two or more inhibitors of components of super-enhancers described herein in a therapeutically effective amount to a subject in need thereof. A subject may be a human, non-human primate, rodent, canine, feline, ungulate, bovine, equine, or other species.

Accordingly, the present invention is directed to a pharmaceutical composition comprising the following therapeutic agents: (i) alvocidib, or a pharmaceutically acceptable salt thereof; and (ii) a bromodomain (BRD) inhibitor, or a pharmaceutically acceptable salt thereof; for use in a method for treatment of cancer in a mammal in need thereof.

The claimed therapeutic agents can be co-administered or administered sequentially. For example, a first therapeutic agent can be administered and after a sufficient period of time a second therapeutic agent is administered. One of ordinary skill in the art can derive an appropriate dosing schedule based on common techniques and knowledge.

In certain embodiments, at least one of the therapeutic agents will inhibit cyclin-dependent kinase (Cdk) proteins, such as Cdk4, Cdk6, Cdk7, Cdk8, Cdk9, Cdk10 and/or Cdk11. In some embodiments, the cyclin-dependent kinase inhibitor inhibits Cdk7, Cdk9 or both. According to the invention, at least one of the therapeutic agents is alvocidib (Int. J. Oncol. 1996 Dec 9(6): 1143-68).

In other embodiments, at least one of the therapeutic agents will inhibit bromodomain proteins such as Brd2, Brd3, Brd4 and/or BrdT, for example Brd4. In some of these embodiments, the therapeutic agent is BI2536 (ACS Chem. Biol. 2014 May 16;9(5):1160-71; Boehringer Ingelheim), TG101209 (ACS Chem. Biol. 2014 May 16;9(5):1160-71), OTX015 (Mol. Cancer Ther. November 201312; C244; Oncoethix), IBET762 (J Med Chem. 2013 Oct 10;56(19):7498-500; GlaxoSmithKline), IBET151 (Bioorg. Med. Chem. Lett. 2012 Apr 15;22(8):2968-72; GlaxoSmithKline), PFI-1 (J. Med. Chem. 2012 Nov 26;55(22):9831-7; Cancer Res. 2013 Jun 1;73(11):3336-46; Structural Genomics Consortium) or CPI-0610 (Constellation Pharmaceuticals).

In some other embodiments, the method comprises administering to the mammal an effective amount of a cyclin-dependent kinase inhibitor and a bromodomain inhibitor. For example, in some embodiments the cyclin-dependent kinase inhibitor is alvocidib. In other different embodiments of the foregoing the bromodomain inhibitor is IBET762. In still more embodiments of the foregoing the bromodomain inhibitor is OTX015.

In other embodiments, the method comprises administering to the mammal an effective amount of alvocidib and an effective amount of IBET762. In still more embodiments, the method comprises administering to the mammal an effective amount of alvocidib and an effective amount of OTX015.

A wide variety of cancers, including solid tumors and leukemias (e.g., acute myeloid leukemia) are amenable to the methods disclosed herein. Types of cancer that may be treated in various embodiments include, but are not limited to: adenocarcinoma of the breast, prostate, and colon; all forms of bronchogenic carcinoma of the lung; myeloid; melanoma; hepatoma; neuroblastoma; papilloma; apudoma; choristoma; branchioma; malignant carcinoid syndrome; carcinoid heart disease; and carcinoma (e.g., Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell). Additional types of cancers that may be treated include: histiocytic disorders; leukemia; histiocytosis malignant; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor. Further, the following types of cancers are also contemplated as amenable to treatment: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin. The types of cancers that may be treated also include, but are not limited to, angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

### II. PHARMACEUTICAL COMPOSITIONS

Furthermore, the present disclosure is directed to pharmaceutical compositions. The pharmaceutical composition comprises (i) alvocidib, or a pharmaceutically acceptable salt thereof; and (ii) a bromodomain (BRD) inhibitor, or a pharmaceutically acceptable salt thereof.

In other specific embodiments, the pharmaceutical composition comprises a cyclin-dependent kinase inhibitor and a bromodomain inhibitor. For example, in some of these embodiments the cyclin-dependent kinase inhibitor is alvocidib. In other different embodiments of the foregoing the bromodomain inhibitor is IBET762. In in still more embodiments of the foregoing the bromodomain inhibitor is OTX015.

In other embodiments, the pharmaceutical composition comprises a pharmaceutically acceptable carrier or excipient, alvocidib and IBET762. In still more embodiments, the pharmaceutical composition comprises a pharmaceutically acceptable carrier or excipient, alvocidib and OTX015.

In some embodiments, the pharmaceutical composition is formulated for oral administration. In other embodiments, the pharmaceutical composition is formulated for injection.

Suitable routes of administration include, but are not limited to, oral, intravenous, rectal, aerosol, parenteral, ophthalmic, pulmonary, transmucosal, transdermal, vaginal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

In certain embodiments, a compound as described herein is administered in a local rather than systemic manner, for example, via injection of the compound directly into an organ, often in a depot preparation or sustained release formulation. In specific embodiments, long acting formulations are administered by implantation (for example subcutaneously or intramuscularly) or by intramuscular injection. Furthermore, in other embodiments, the drug is delivered in a targeted drug delivery system, for example, in a liposome coated with organ-specific antibody. In such embodiments, the liposomes are targeted to and taken up selectively by the organ. In yet other embodiments, the compound as described herein is provided in the form of a rapid release formulation, in the form of an extended release formulation, or in the form of an intermediate release formulation. In yet other embodiments, the compound described herein is administered topically.

The compounds according to the invention are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that are used in some embodiments. An exemplary dosage is 10 to 30 mg per day. The exact dosage will depend upon the route of administration, the form in which the compound is administered, the subject to be treated, the body weight of the subject to be treated, and the preference and experience of the attending physician.

In some embodiments, a compound of the invention is administered in a single dose. Typically, such administration will be by injection, e.g., intravenous injection, in order to introduce the agent quickly. However, other routes are used as appropriate. A single dose of a compound of the invention may also be used for treatment of an acute condition.

In some embodiments, a compound of the invention is administered in multiple doses. In some embodiments, dosing is about once, twice, three times, four times, five times, six times, or more than six times per day. In other embodiments, dosing is about once a month, once every two weeks, once a week, or once every other day. In another embodiment a compound of the invention and another agent are administered together about once per day to about 6 times per day. In another embodiment the administration of a compound of the invention and an agent continues for less than about 7 days. In yet another embodiment the administration continues for more than about 6, 10, 14, 28 days, two months, six months, or one year. In some cases, continuous dosing is achieved and maintained as long as necessary.

Administration of the compounds of the invention may continue as long as necessary. In some embodiments, a compound of the invention is administered for more than 1, 2, 3, 4, 5, 6, 7, 14, or 28 days. In some embodiments, a compound of the invention is administered for less than 28, 14, 7, 6, 5, 4, 3, 2, or 1 day. In some embodiments, a compound of the invention is administered chronically on an ongoing basis, e.g., for the treatment of chronic effects.

In some embodiments, the compounds of the invention are administered in dosages. Due to intersubject variability in compound pharmacokinetics, individualization of dosing regimen is provided in certain embodiments. Dosing for a compound of the invention may be found by routine experimentation in light of the instant disclosure and/or can be derived by one of ordinary skill in the art.

In some embodiments, the compounds described herein are formulated into pharmaceutical compositions. In specific embodiments, pharmaceutical compositions are formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

Provided herein are pharmaceutical compositions comprising inhibitors targeting at least two super-enhancer components selected from the following i) cyclin-dependent kinases (Cdk); ii) bromodomain and extraterminal (BET) subfamily of bromodomain proteins; and a pharmaceutically acceptable diluent(s), excipient(s), or carrier(s). Encompassed herein are all combinations of actives set forth in the combination therapies section below and throughout this disclosure.

A pharmaceutical composition, as used herein, refers to a mixture of inhibitors targeting at least two super-enhancer components with other chemical components, such as carriers, stabilizers, diluents, dispersing agents, suspending agents, thickening agents, and/or excipients. In certain embodiments, the pharmaceutical composition facilitates administration of the compound to an organism. In some embodiments, practicing the methods of treatment or use provided herein, therapeutically effective amounts of two or more inhibitors targeting at least two super-enhancer components provided herein are administered in a pharmaceutical composition to a mammal having a disease, disorder or medical condition to be treated. In specific embodiments, the mammal is a human. In certain embodiments, therapeutically effective amounts vary depending on the severity of the disease, the age and relative health of the subject, the potency of the compound used and other factors. The compounds described herein are used singly or in combination with one or more therapeutic agents as components of mixtures.

In one embodiment, inhibitors targeting at least two super-enhancer components are formulated in an aqueous solution. In specific embodiments, the aqueous solution is selected from, by way of example only, a physiologically compatible buffer, such as Hank's solution, Ringer's solution, or physiological saline buffer. In other embodiments, inhibitors targeting at least two super-enhancer components are formulated for transmucosal administration. In specific embodiments, transmucosal formulations include penetrants that are appropriate to the barrier to be permeated. In still other embodiments wherein the compounds described herein are formulated for other parenteral injections, appropriate formulations include aqueous or nonaqueous solutions. In specific embodiments, such solutions include physiologically compatible buffers and/or excipients.

In another embodiment, compounds described herein are formulated for oral administration. Compounds described herein are formulated by combining the active compounds with, e.g., pharmaceutically acceptable carriers or excipients. In various embodiments, the compounds described herein are formulated in oral dosage forms that include, by way of example only, tablets, powders, pills, dragees, capsules, liquids, gels, syrups, elixirs, slurries, suspensions and the like.

In certain embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In specific embodiments, disintegrating agents are optionally added. Disintegrating agents include, by way of example only, cross-linked croscarmellose sodium, polyvinylpyrrolidone, agar, or alginic acid or a salt thereof such as sodium alginate.

In one embodiment, dosage forms, such as dragee cores and tablets, are provided with one or more suitable coating. In specific embodiments, concentrated sugar solutions are used for coating the dosage form. The sugar solutions, optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In certain embodiments, therapeutically effective amounts of inhibitors targeting at least two super-enhancer components described herein are formulated into other oral dosage forms. Oral dosage forms include push-fit capsules made of gelatin, as well as soft, sealed capsules made of gelatin and a plasticizer, such as glycerol or sorbitol. In specific embodiments, push-fit capsules contain the active ingredients in admixture with one or more filler. Fillers include, by way of example only, lactose, binders such as starches, and/or lubricants such as talc or magnesium stearate and, optionally, stabilizers. In other embodiments, soft capsules, contain one or more active compound that is dissolved or suspended in a suitable liquid. Suitable liquids include, by way of example only, one or more fatty oil, liquid paraffin, or liquid polyethylene glycol. In addition, stabilizers are optionally added.

In other embodiments, therapeutically effective amounts of inhibitors targeting at least two super-enhancer components described herein are formulated for buccal or sublingual administration. Formulations suitable for buccal or sublingual administration include, by way of example only, tablets, lozenges, or gels. In still other embodiments, the compounds described herein are formulated for parental injection, including formulations suitable for bolus injection or continuous infusion. In specific embodiments, formulations for injection are presented in unit dosage form (e.g., in ampoules) or in multi-dose containers. Preservatives are, optionally, added to the injection formulations. In still other embodiments, the pharmaceutical compositions are formulated in a form suitable for parenteral injection as sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active compounds in water-soluble form. In additional embodiments, suspensions of the active compounds (e.g., compounds of structure (I)) are prepared as appropriate oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In certain specific embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the compounds to allow for the preparation of highly concentrated solutions. Alternatively, in other embodiments, the active ingredient is in powder form for constitution with a suitable vehicle, e.g., sterile pyrogen-free water, before use.

In still other embodiments, inhibitors targeting at least two super-enhancer components are administered topically. The compounds described herein are formulated into a variety of topically administrable compositions, such as solutions, suspensions, lotions, gels, pastes, medicated sticks, balms, creams or ointments. Such pharmaceutical compositions optionally contain solubilizers, stabilizers, tonicity enhancing agents, buffers and preservatives.

In yet other embodiments, inhibitors targeting at least two super-enhancer components are formulated for transdermal administration. In specific embodiments, transdermal formulations employ transdermal delivery devices and transdermal delivery patches and can be lipophilic emulsions or buffered, aqueous solutions, dissolved and/or dispersed in a polymer or an adhesive. In various embodiments, such patches are constructed for continuous, pulsatile, or on demand delivery of pharmaceutical agents. In additional embodiments, the transdermal delivery of inhibitors targeting at least two super-enhancer components is accomplished by means of iontophoretic patches and the like. In certain embodiments, transdermal patches provide controlled delivery of inhibitors targeting at least two super-enhancer components. In specific embodiments, the rate of absorption is slowed by using rate-controlling membranes or by trapping the compound within a polymer matrix or gel. In alternative embodiments, absorption enhancers are used to increase absorption. Absorption enhancers or carriers include absorbable pharmaceutically acceptable solvents that assist passage through the skin. For example, in one embodiment, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin.

In other embodiments, inhibitors targeting at least two super-enhancer components are formulated for administration by inhalation. Various forms suitable for administration by inhalation include, but are not limited to, aerosols, mists or powders. Pharmaceutical compositions of inhibitors targeting at least two super-enhancer components are conveniently delivered in the form of an aerosol spray presentation from pressurized packs or a nebulizer, with the use of a suitable propellant (e.g., dichlorodifluoromethane, trichlorofluoromethane, dichlorotetrafluoroethane, carbon dioxide or other suitable gas). In specific embodiments, the dosage unit of a pressurized aerosol is determined by providing a valve to deliver a metered amount. In certain embodiments, capsules and cartridges of, such as, by way of example only, gelatin for use in an inhaler or insufflator are formulated containing a powder mix of the compound and a suitable powder base such as lactose or starch.

In still other embodiments, inhibitors targeting at least two super-enhancer components are formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone, PEG, and the like. In suppository forms of the compositions, a low-melting wax such as, but not limited to, a mixture of fatty acid glycerides, optionally in combination with cocoa butter is first melted.

In certain embodiments, pharmaceutical compositions are formulated in any conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are optionally used as suitable. Pharmaceutical compositions comprising inhibitors targeting at least two super-enhancer components are manufactured in a conventional manner, such as, by way of example only, by means of conventional mixing, dissolving, granulating, dragee-making, levigating, emulsifying, encapsulating, entrapping or compression processes.

Pharmaceutical compositions include at least one pharmaceutically acceptable carrier, diluent or excipient and inhibitors targeting at least two super-enhancer components, described herein as an active ingredient. The active ingredient is in free-acid or free-base form, or in a pharmaceutically acceptable salt form. In addition, the methods and pharmaceutical compositions described herein include the use of N-oxides, crystalline forms (also known as polymorphs), as well as active metabolites of these compounds having the same type of activity. All tautomers of the compounds described herein are included within the scope of the compounds presented herein. Additionally, the compounds described herein encompass unsolvated as well as solvated forms with pharmaceutically acceptable solvents such as water, ethanol, and the like. The solvated forms of inhibitors targeting at least two super-enhancer components presented herein are also considered to be disclosed herein. In addition, the pharmaceutical compositions optionally include other medicinal or pharmaceutical agents, carriers, adjuvants, such as preserving, stabilizing, wetting or emulsifying agents, solution promoters, salts for regulating the osmotic pressure, buffers, and/or other therapeutically valuable substances.

Methods for the preparation of compositions comprising inhibitors targeting at least two super-enhancer components described herein include formulating the compounds with one or more inert, pharmaceutically acceptable excipients or carriers to form a solid, semi-solid or liquid. Solid compositions include, but are not limited to, powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein. Semi-solid compositions include, but are not limited to, gels, suspensions and creams. The form of the pharmaceutical compositions described herein include liquid solutions or suspensions, solid forms suitable for solution or suspension in a liquid prior to use, or as emulsions. These compositions also optionally contain minor amounts of nontoxic, auxiliary substances, such as wetting or emulsifying agents, pH buffering agents, and so forth.

In some embodiments, pharmaceutical composition comprising inhibitors targeting at least two super-enhancer components illustratively takes the form of a liquid where the agents are present in solution, in suspension or both. Typically when the composition is administered as a solution or suspension a first portion of the agent is present in solution and a second portion of the agent is present in particulate form, in suspension in a liquid matrix. In some embodiments, a liquid composition includes a gel formulation. In other embodiments, the liquid composition is aqueous.

In certain embodiments, useful aqueous suspensions contain one or more polymers as suspending agents. Useful polymers include water-soluble polymers such as cellulosic polymers, e.g., hydroxypropyl methylcellulose, and water-insoluble polymers such as cross-linked carboxyl-containing polymers. Certain pharmaceutical compositions described herein comprise a mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Useful pharmaceutical compositions also, optionally, include solubilizing agents to aid in the solubility of inhibitors targeting at least two super-enhancer components. The term "solubilizing agent" generally includes agents that result in formation of a micellar solution or a true solution of the agent. Certain acceptable nonionic surfactants, for example polysorbate 80, are useful as solubilizing agents, as can ophthalmically acceptable glycols, polyglycols, e.g., polyethylene glycol 400, and glycol ethers.

Furthermore, useful pharmaceutical compositions optionally include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Additionally, useful compositions also, optionally, include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Other useful pharmaceutical compositions optionally include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

Still other useful compositions include one or more surfactants to enhance physical stability or for other purposes. Suitable nonionic surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

Still other useful compositions include one or more antioxidants to enhance chemical stability where required. Suitable antioxidants include, by way of example only, ascorbic acid and sodium metabisulfite.

In certain embodiments, aqueous suspension compositions are packaged in single-dose non-reclosable containers. Alternatively, multiple-dose reclosable containers are used, in which case it is typical to include a preservative in the composition.

In alternative embodiments, other delivery systems for hydrophobic pharmaceutical compounds are employed. Liposomes and emulsions are examples of delivery vehicles or carriers useful herein. In certain embodiments, organic solvents such as N-methylpyrrolidone are also employed. In additional embodiments, the compounds described herein are delivered using a sustained-release system, such as semipermeable matrices of solid hydrophobic polymers containing the therapeutic agent. Various sustained-release materials are useful herein. In some embodiments, sustained-release capsules release the compounds for a few weeks up to over 100 days. Depending on the chemical nature and the biological stability of the therapeutic reagent, additional strategies for protein stabilization are employed.

In certain embodiments, the formulations described herein comprise one or more antioxidants, metal chelating agents, thiol containing compounds and/or other general stabilizing agents. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

In some embodiments, the concentration of one or more inhibitors provided in the pharmaceutical compositions of the present invention is less than 100%, 90%, 80%, 70%, 60%, 50%, 40%, 30%, 20%, 19%, 18%, 17%, 16%, 15%,14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v or v/v.

In some embodiments, the concentration of one or more inhibitors of the invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125% , 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v, or v/v.

In some embodiments, the concentration of one or more inhibitors of the invention is in the range from approximately 0.0001% to approximately 50%, approximately 0.001% to approximately 40 %, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v.

In some embodiments, the concentration of one or more inhibitors of the invention is in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v.

In some embodiments, the amount of one or more inhibitors of the invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of one or more inhibitors of the invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g,, 0.15 g, 0.2 g,, 0.25 g, 0.3 g,, 0.35 g, 0.4 g,, 0.45 g, 0.5 g, 0.55 g, 0.6 g,, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5g, 7 g, 7.5g, 8 g, 8.5 g, 9 g, 9.5 g, or 10 g.

In some embodiments, the amount of one or more inhibitors of the invention is in the range of 0.0001-10 g, 0.0005-9 g, 0.001-8 g, 0.005-7 g, 0.01-6 g, 0.05-5 g, 0.1-4 g, 0.5-4 g, or 1-3 g.

### EXAMPLES

### EXAMPLE 1

### COMBINATION TREATMENT EFFECT ON TUMOR VOLUME

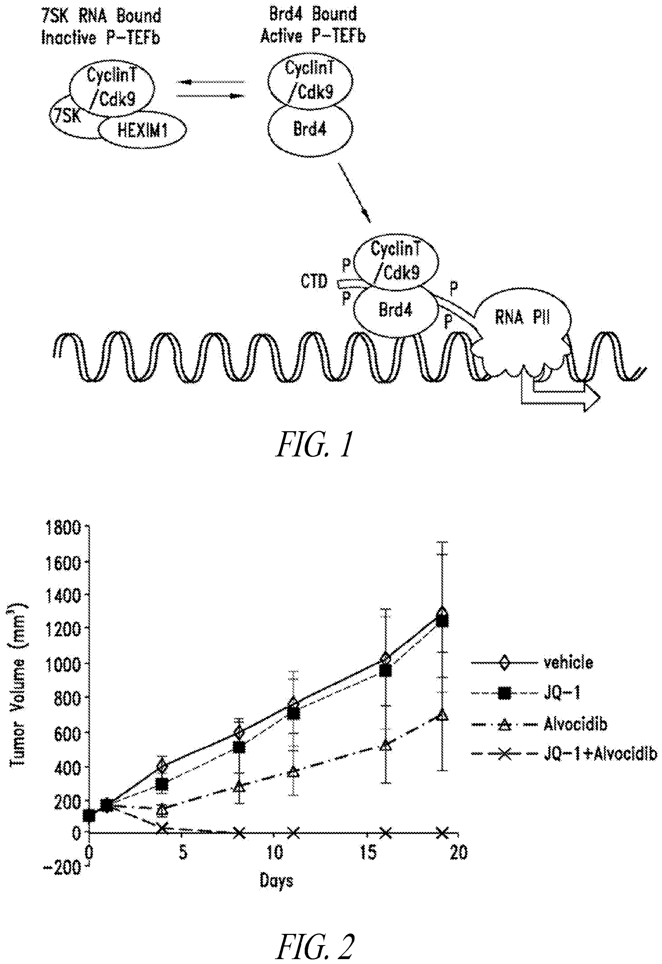

The AML cell line, MV4-11 is MLL fusion and FLT3-ITD positive. The MLL fusion makes this cell line particularly dependent upon super-enhancer function for driving oncogenesis. MV4-11 cells were implanted subcutaneously in athymic Nu/Nu mice and allowed to establish until tumors reached 100-200 mm³. JQ-1, a BET protein inhibitor, and alvocidib, a Cdk inhibitor, were used alone and in combination in order to determine the effect of the drug(s) on tumor volume. Animals were stratified into 4 groups and administered one of four treatments: vehicle, 25 mg/kg JQ-1, 2.5 mg/kg alvocidib, or a combination of the two drugs at these doses. The drugs were administered intraperitoneally, once daily for 5 days, for a total of 3 weeks. Tumor volume was measured twice weekly by caliper measurement. The results are shown in Figure 2. JQ-1 at 25 mg/kg showed no measurable activity, as it tracked closely with the vehicle control. Alvocidib at 2.5 mg/kg showed moderate activity with the tumors being approximately 50% the size of the vehicle throughout the study. Surprisingly, the combination of these two agents at these dose levels completely stopped tumor growth in these mice by day 8. No tumors were detected in any of the mice (n=8) at the completion of the study in the combination group. Body weight for all animals was comparable throughout the study. Weight gain in vehicle and individual drug-treated mice could be attributed to tumor growth. Weight gain in the mice given the combination treatment could be attributed to the overall health and food intake of the mice.

These results are strong evidence for synergy between alvocidib and JQ-1 and also to the concept of targeting the super-enhancer complex at two or more points.

### EXAMPLE 2

### COMBINATION TREATMENT EFFECT ON C-MYC EXPRESSION

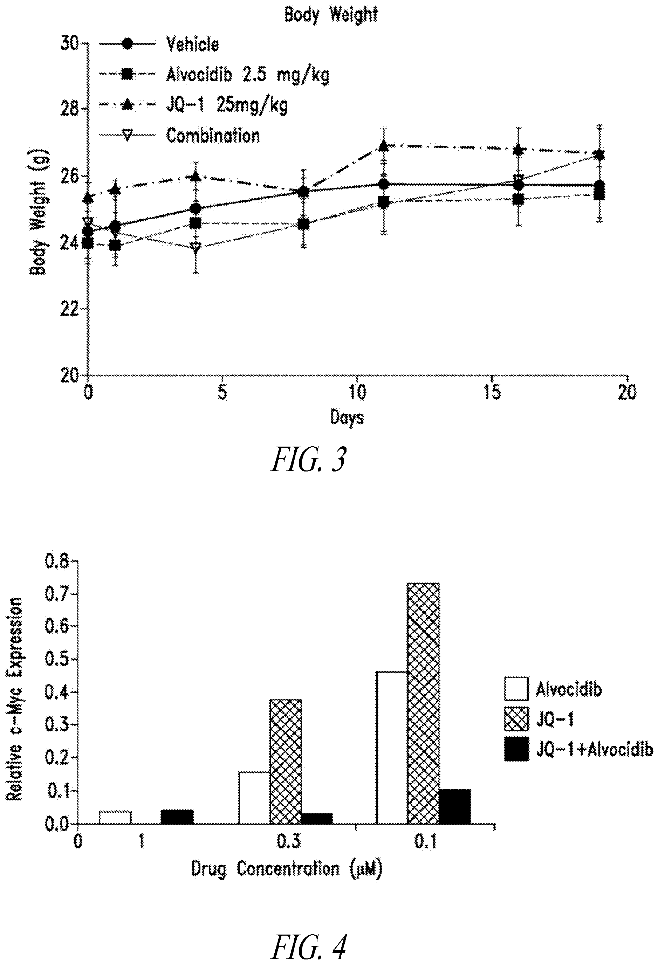

The c-MYC gene is well known to be regulated by the super-enhancer complex in AML cells. JQ-1, a BET protein inhibitor, and alvocidib, a Cdk inhibitor, were used alone and in combination in order to determine the effect of the drug(s) on c-MYC expression. MV4-11 cells were treated with various concentrations of alvocidib and/or JQ-1 for 2 hours. DMSO-treated controls were also included. Treatment was stopped by lysing the cells and isolating RNA for RT-qPCR analysis. The expression of c-MYC was determined and normalized to the housekeeping gene, HPRT1 and to the DMSO-treated control. The results are shown in Figure 3. Alvocidib and JQ-1 downregulated the expression of c-MYC when used alone; however, the downregulation of c-MYC was much more profound when the two agents were used in combination. Again, these data demonstrate strong synergy between these two agents and provide good rationale for the approach of targeting the super-enhancer complex through multiple mechanisms.

### EXAMPLE 3

### COMBINATION TREATMENT EFFECT ON IC₅₀

MOLM13 and MV4-11 are MLL fusion and FLT3-ITD positive, AML cells lines. Both cell lines were treated with 20 nM of alvocidib, a Cdk inhibitor, for 48 hours or DMSO control, followed by the addition of JQ-1, an inhibitor of BET proteins, at various concentrations (10 µM - 0.0003 µM) or by the addition of panobinostat, an inhibitor of HDAC, at various concentrations (10 µM - 0.0003 µM) for 48 additional hours. At completion, cell viability was measured by Cell Titer GLO reagent. IC₅₀ values were determined for JQ-1 and panobinostat with and without the low dose of alvocidib. The results are shown in Table 1 below. Addition of 20 nM alvocidib increased the potency of JQ-1, as shown by an IC₅₀ value of 2-fold lower. Addition of 20 nM alvocidib increased the potency of panobinostat (an HDAC inhibitor), as demonstrated by IC₅₀ values of 3- to 6-fold lower. These data demonstrate increased activity for the combined Cdk and BET protein inhibitors and Cdk and HDAC inhibitors, relative to the individual inhibitors.

**Table 1**

| Alvocidib lowers IC₅₀ of JQ-1 and Panobinostat | | | | |
|---|---|---|---|---|
| **Alvocidib** | **JQ-1** | | **Panobinostat** | |
| + | - | + | - | + |
| MOLM13 | 526 nM | 209 nM | 16 nM | 5 nM |
| MV4-11 | 228 nM | 133 nM | 18 nM | 3 nM |

### EXAMPLE 4

### INHIBITION OF MYC AND MCL-1 EXPRESSION BY ALVOCIDIB

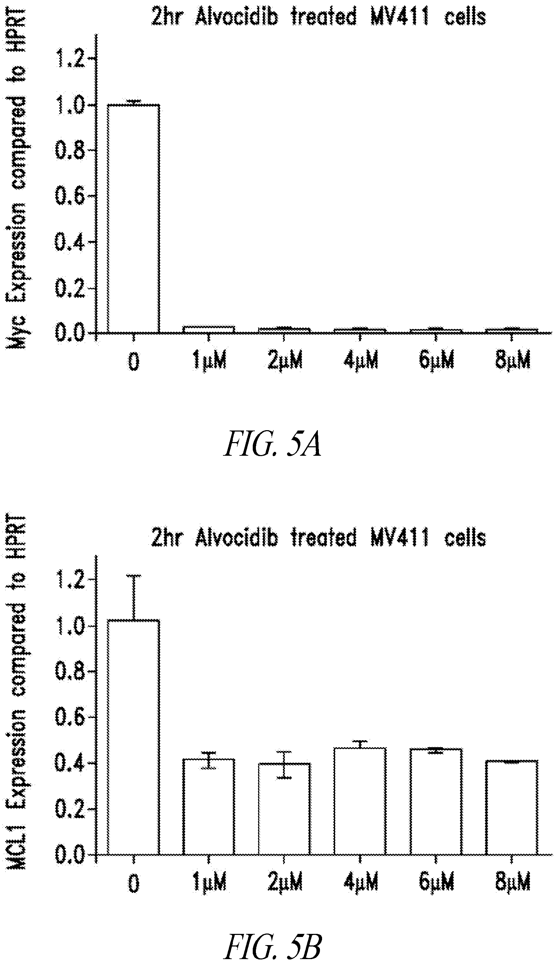

To determine the MYC and MCL-1 inhibitory activity of alvocidib, MV4-11 cells were treated with alvocidib for two hours and the expression of MYC and MCL-1 relative to HPRT was determined. The data show that alvocidib as a single agent completely eliminates MYC expression in MV4-11 cells (Figure 5A) and reduces MCL-1 expression by 60% (Figure 5B).

### EXAMPLE 5

### ALVOCIDIB COMBINED WITH BRD4 INHIBITORS INCREASES APOPTOSIS IN MV4-11 CELLS

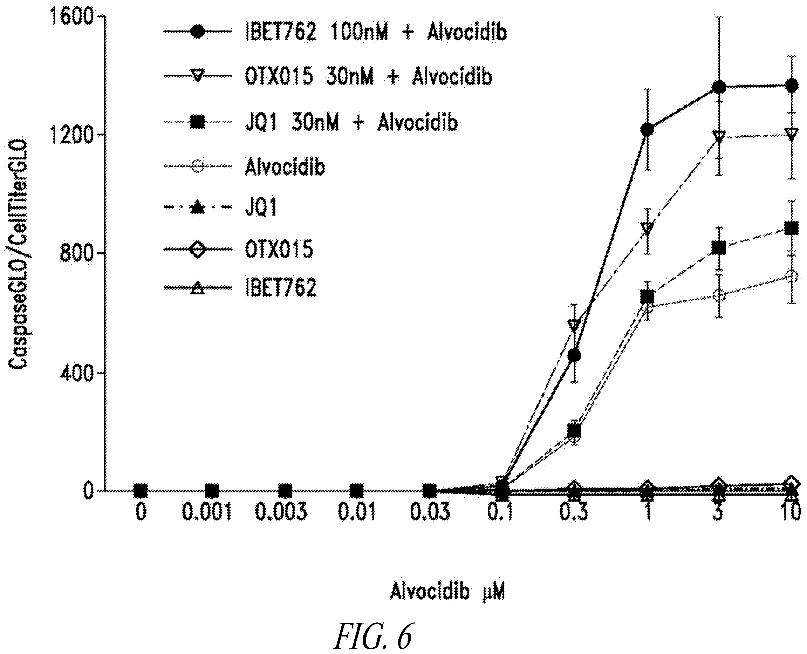

MV4-11 cells were seeded at 1,000 cells per well in 384 well plates. BRD4 inhibitor (IBET762, OTX015 or JQ1) was added at a single dose to cells for 24hr. Alvocidib was added at 24hr in a dose dilution. Cell Titer GLO and Caspase GLO were added and luminescence was measured at 48hr. Single doses of BRD4 inhibitors combined with alvocidib in a serial dilution demonstrated a synergistic increase in caspase activity, indicating that alvocidib combined with BRD4 inhibitors synergistically increases apoptosis in MV4-11 cancer cells when compared with alvocidib or individual BRD4 inhibitors alone (Figure 6).

### EXAMPLE 6

### ALVOCIDIB COMBINED WITH BRD4 INHIBITORS REDUCES MYC AND MCL-1 EXPRESSION IN MV4-11 CELLS BETTER THAN INDIVIDUAL TREATMENTS

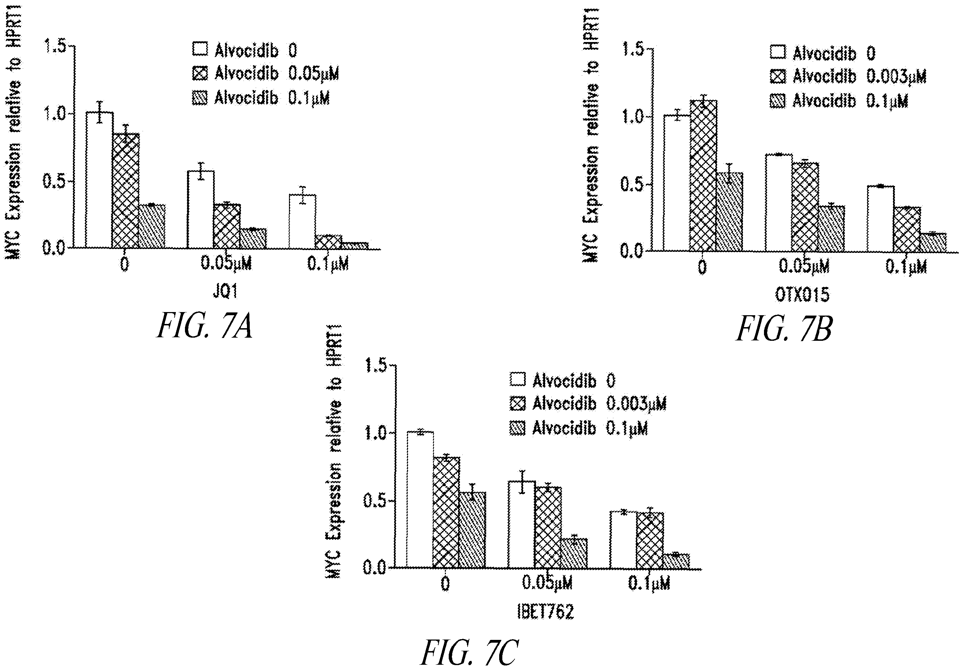

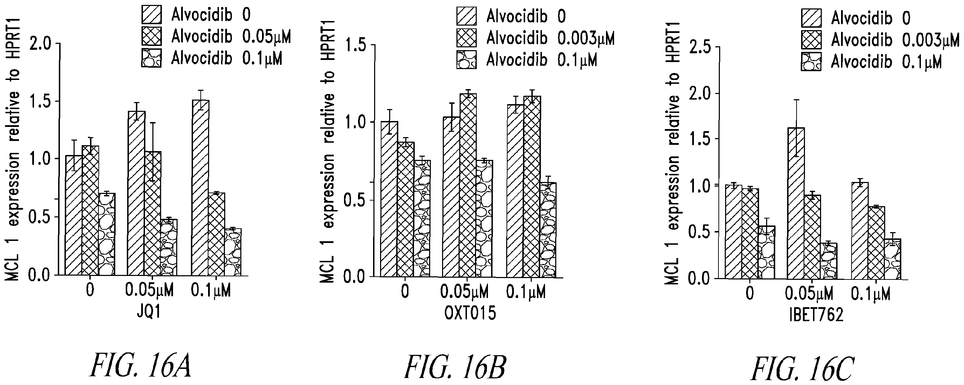

MV4-11 cells were seeded (1 million cells) 6-well plates and treated with alvocidib or a BRD4 inhibitor (IBET762, OTX015 or JQ1) alone or treated with a combination of alvocidib and a BDR4 inhibitor (IBET762, OTX015 or JQ1). After treatment cells were analyzed by RT-PCR. The combination of alvocidib and a BRD4 inhibitor reduced MYC expression better than individual treatments (Figure 7A-C). The combination of alvocidib and a BRD4 inhibitor also reduced MCL-1 expression better than individual treatments (Figure 16A-C).

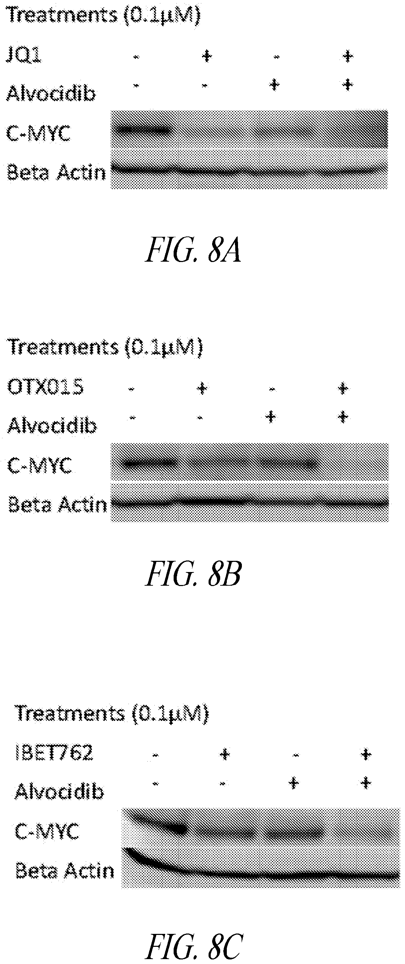

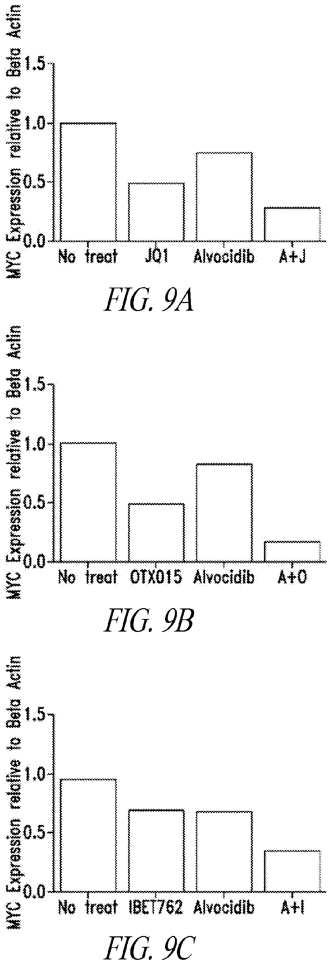

In a related experiment, MV4-11 cells were seeded (3 million cells) in T75 flasks and treated with alvocidib or a BRD4 inhibitor (IBET762, OTX015 or JQ1) alone or treated with a combination of alvocidib and a BDR4 inhibitor (IBET762, OTX015 or JQ1) for 3 hr. Cells were analyzed by western blotting which showed a decrease in MYC expression for all treated samples (Figures 8A-C). Quantification of western blots shows that the combination of alvocidib (0.1mM) and a BRD4 inhibitor (0.1mM) reduces MYC expression better than individual treatments (Figs. 9A-C).

### EXAMPLE 7

### ALVOCIDIB INCREASES ASSOCIATION OF CDK9 AND HEXIM1 PROTEINS IN A TIME AND DOSE DEPENDENT MANNER

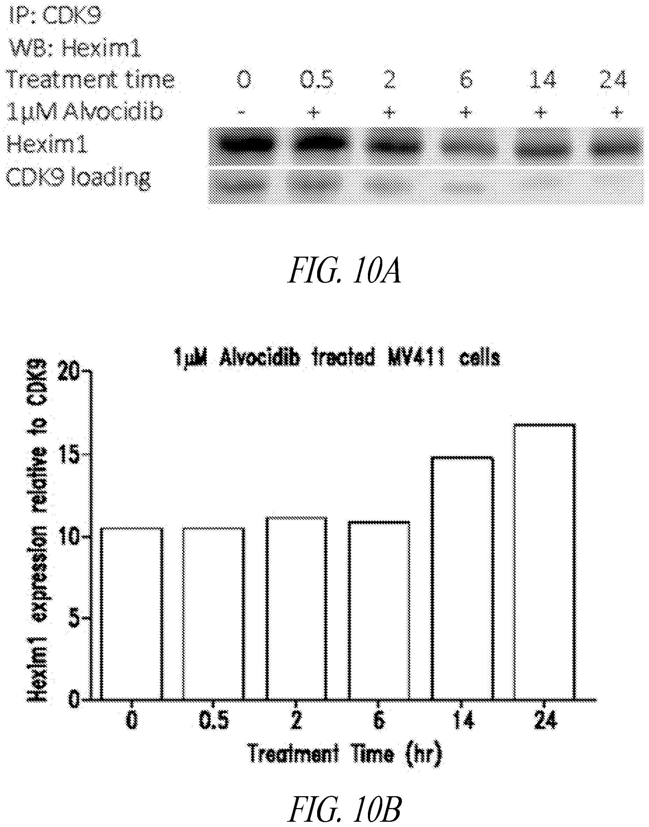

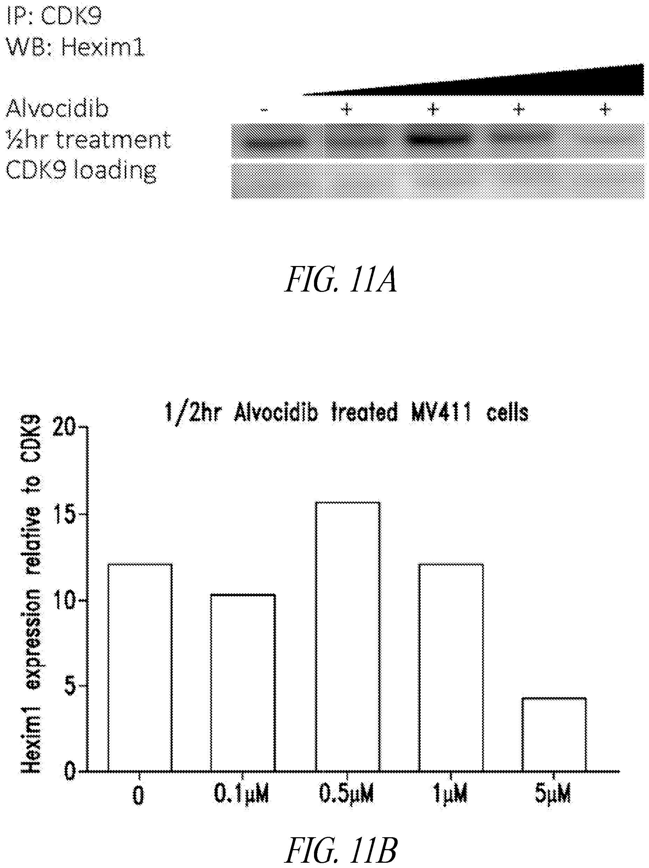

MV4-11 cells were seeded and treated with alvocidib in identical concentrations and doses. Cells were collected at given times and subjected to high salt lysis buffer and immunoprecipitation followed by western blotting. The data show that 1mM alvocidib increases association of CDK9 and Hexim1 in a time dependent manner. A representative western blot is provided in Figure 10A, and quantification of the western blot is shown in Figure 10B. Figures 11A and 11B provide results of a dose escalation study performed according to the above general procedure. The data show that different doses of alvocidib alter CDK9 and Hexim1 association at the half hour time point. A 500nM dose of alvocidib demonstrated an increased CDK9/Hexim1 association. Representative western blot is provided in Figure 11A with quantification of the western blot in Figure 11B.

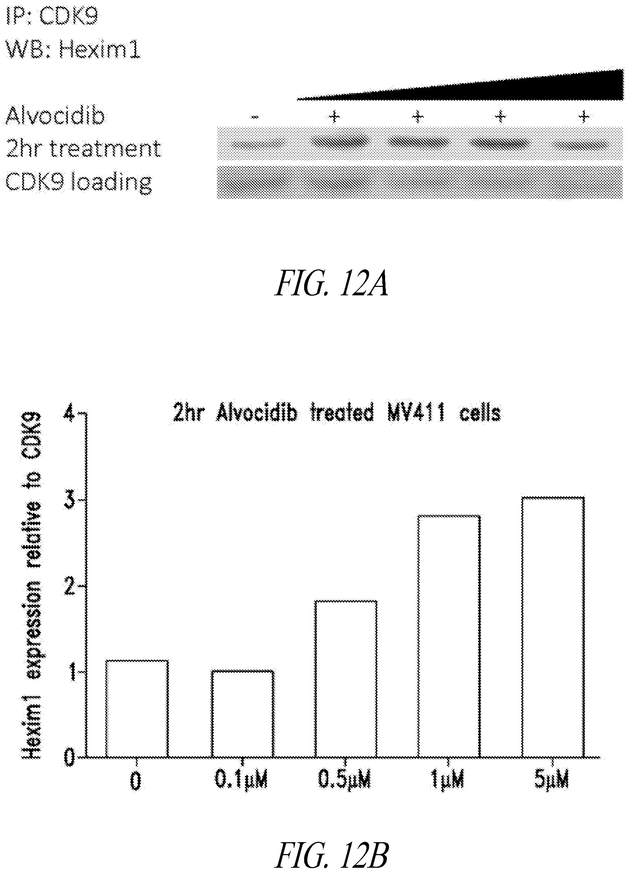

Similar experiments show that a 2hr treatment with different doses of alvocidib demonstrates a dose dependent increase in CDK9/Hexim1 association. Representative western blot is provided in Figure 12A with quantification of the western blot in Figure 12B.

### EXAMPLE 8

### ALVOCIDIB REPRESSES SUPER ENHANCER COMPLEX REGULATED GENES IN A TIME AND DOSE DEPENDENT MANNER

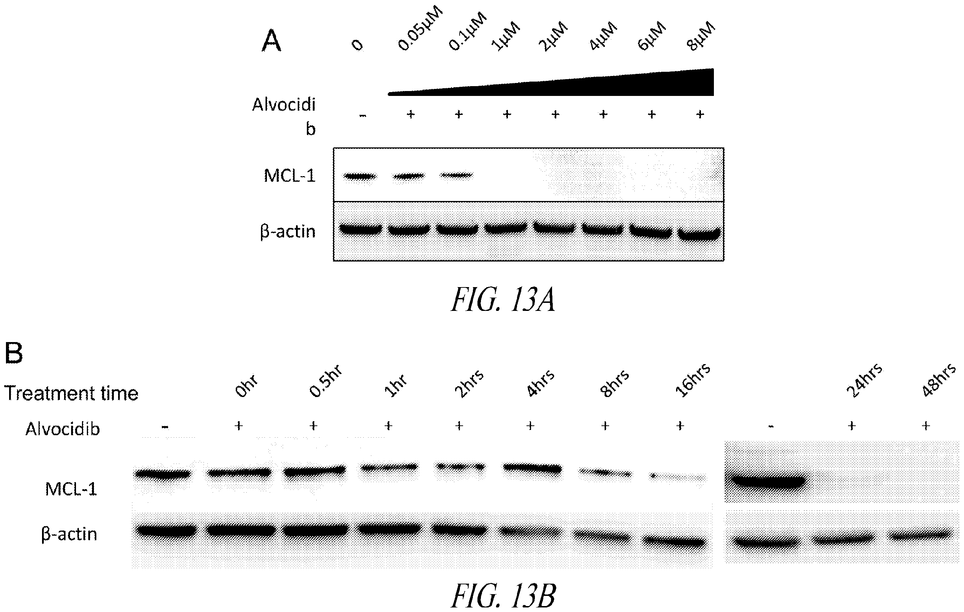

A549 cells are adenocarcinomic human alveolar basal epithelial cells used as a type II pulmonary epithelial model (solid tumor). These cells were seeded and treated with alvocidib in identical concentrations and doses. Treatment was stopped by lysing the cells and isolating RNA for RT-qPCR analysis. The data show that alvocidib represses super enhancer complex regulated genes in solid tumor cells in a time dependent manner. A representative western blot is provided in Figure 13A. The expression of mRNA was determined and normalized to β-actin and the control. Alvocidib downregulated the expression of mRNA completely at 24 and 48 hour time points.

Figures 13B provide results of a dose escalation study performed according to the procedure provided in this example with cell lysing taking place at 2 hours following treatment with alvocidib. A dosing range (0.05 µM - 8 µM) was used to show mRNA expression and regression responds to alvocidib in a dose dependent manner for A549 cells. The data show that different doses of alvocidib alter mRNA expression at the 2 hour time point with the 0.1 µM dose of alvocidib noticeably repressed. Complete repression was observed in the assay at doses of 1 µM up to 8µM, relative to β-actin and vehicle control.

### EXAMPLE 9

### ALVOCIDIB REPRESSES PROTEIN EXPRESSION IN SOLID TUMOR AND NEUROBLASTOMA CELLS IN A TIME DEPENDENT MANNER

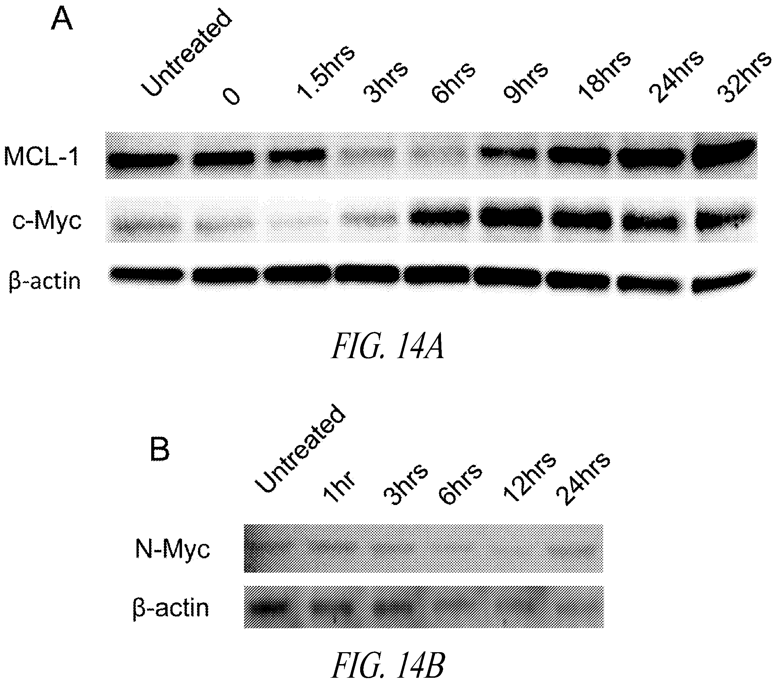

A549 cells and SK-N-AS cells (human adrenal neuroblastoma cell line) were seeded and treated with alvocidib at concentrations of 100 nM and 300 nM respectively. Cells were collected at given times and total protein was extracted and detected using western blotting. The data show that alvocidib represses protein expression of MCL-1 and c-MYC/N-MYC in a time dependent manner through the inhibition of CDK9. A representative western blot is provided in Figures 14A and 14B.

### EXAMPLE 10

### ALVOCIDIB REPRESSES MRNA EXPRESSION IN MV4-11 CELLS IN A TIME DEPENDENT MANNER

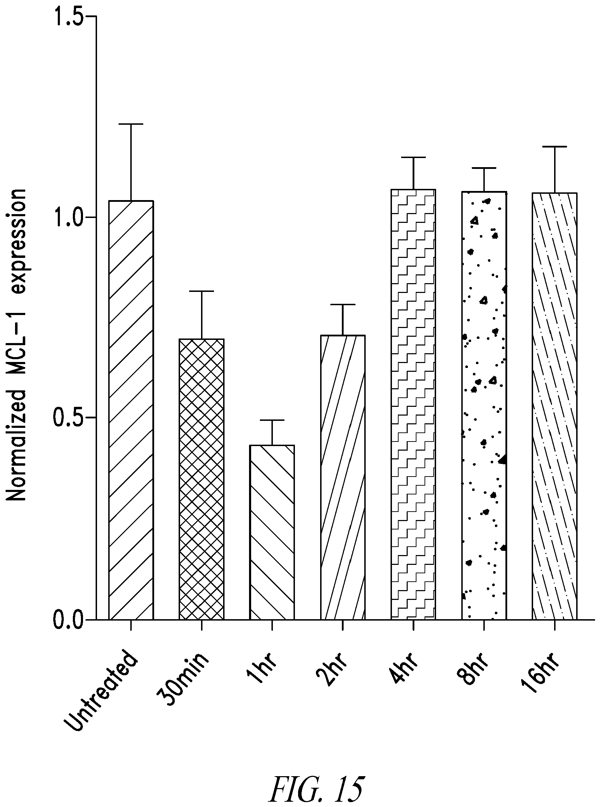

MV4-11 cells were seeded and treated with alvocidib at a concentration of 100 nM. Cells were collected at 0.5, 1, 3, 4, 8, and 16 hours following treatment. Cells were harvested and detection of mRNA was carried out using RT-qPCR analysis in the manner described in previous examples. Expression of MCL-1 was compared to expression of controls and untreated cells. Figure 15 shows alvocidib represses MCL-1 mRNA expression in a time dependent manner. Expression of MCL-1 is repressed at the 30 minute time point, reaches maximum repression at 1 hour post-dose and repression is reduced at the 2 hour time point. There appears to be no inhibition on expression by the 3 hour time point.

### EXAMPLE 11

### ALVOCIDIB COMBINED WITH JQ-1 REDUCES MCL-1 AND C-MYC EXPRESSION IN A549 CELLS BETTER THAN INDIVIDUAL TREATMENTS

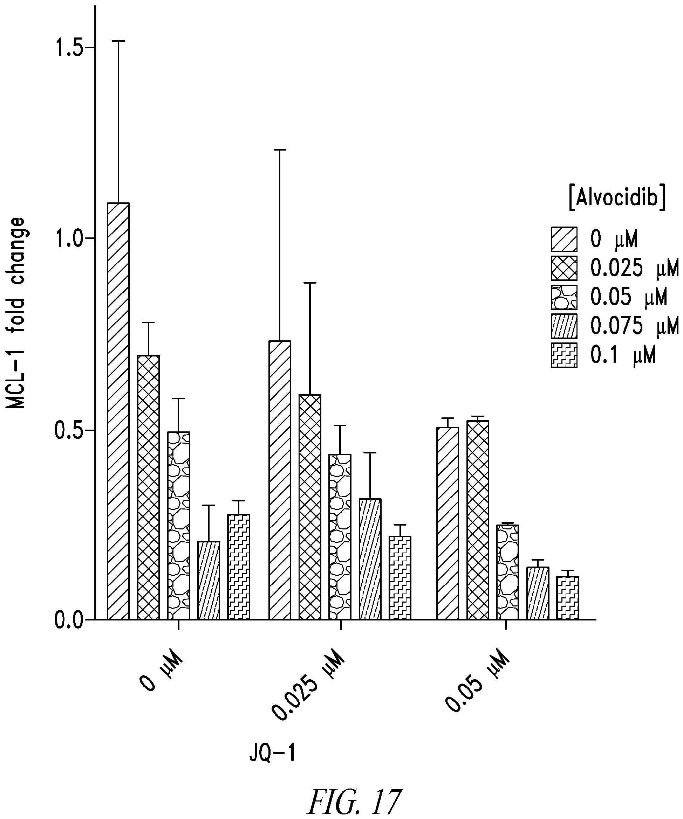

A549 cells were seeded and treated with alvocidib at concentrations ranging from 0 nM - 100 nM in combination with JQ-1 at concentrations ranging from 0 nM - 50 nM. Cells were first treated with JQ-1 for 1 hour followed by treatment with alvocidib for 2 hours. Following the treatment of solid tumor cells, cells were harvested and mRNA was detected using RT-qPCR in the manner described in previous examples. Figure 17 shows alvocidib represses MCL-1 mRNA expression in a time dependent manner through the inhibition of CDK9.

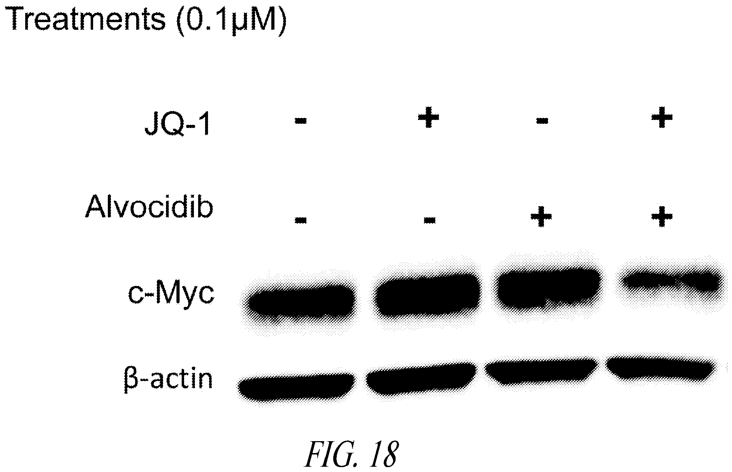

Figure 18 shows that when alvocidib is combined with JQ-1, protein expression of c-MYC is suppressed in A549 cells. Cells were prepared in the same way described within this example, and were then treated for 2 hours with 100 nM alvocidib and 100 nM JQ-1 as indicated in figure 18. Following treatment, cells were harvested and protein detection was carried out using standard immunoblotting techniques.

### EXAMPLE 12

### ALVOCIDIB COMBINED WITH JQ-1 INCREASES APOPTOSIS IN A549 CELLS IN A DOSE DEPENDENT MANNER

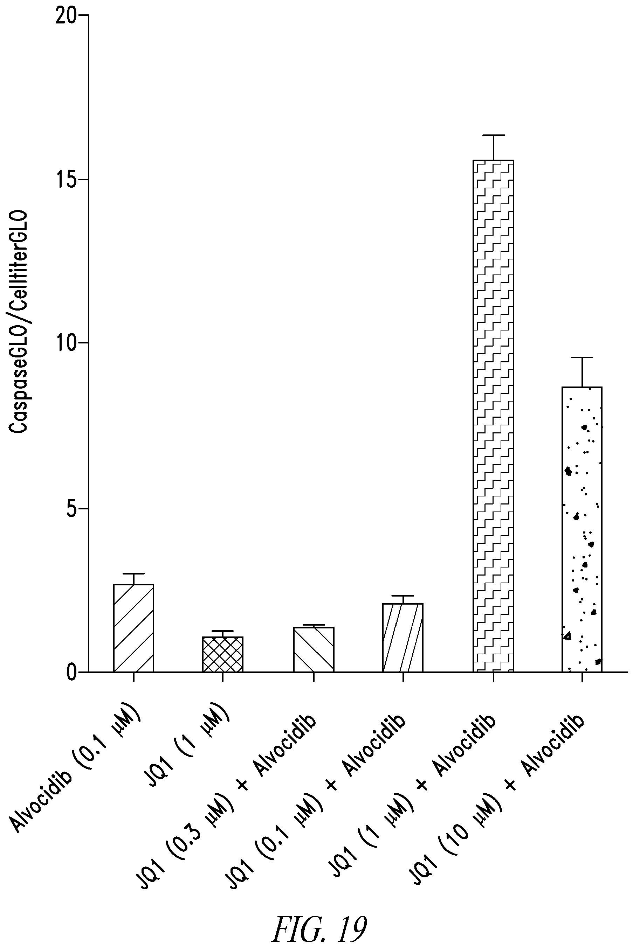

A549 cells were treated with JQ-1 at concentrations ranging from 0.03 - 10 µM for 24 hours (Figure 19). Following the treatment of JQ-1 for 24 hours, alvocidib was added at a concentration of 100 nM. Cell Titer GLO and Caspase GLO were added and luminescence was measured at 48 hours post-dose. Escalating doses of JQ-1 combined with unchanging doses of alvocidib demonstrated a synergistic increase in caspase activity. When a dosing of 1 µM JQ-1 is combined with a dose of 100 nM alvocidib, a greater than 3 fold increase in apoptotic response is observed. This increase indicates alvocidib combined with JQ-1synergistically increases apoptosis in A549 cancer cells when compared with alvocidib or JQ-1 alone (Figure 19).

### EXAMPLE 13

### DINACICLIB COMBINED WITH JQ-1 INCREASES APOPTOSIS IN A549 CELLS IN A DOSE DEPENDENT MANNER

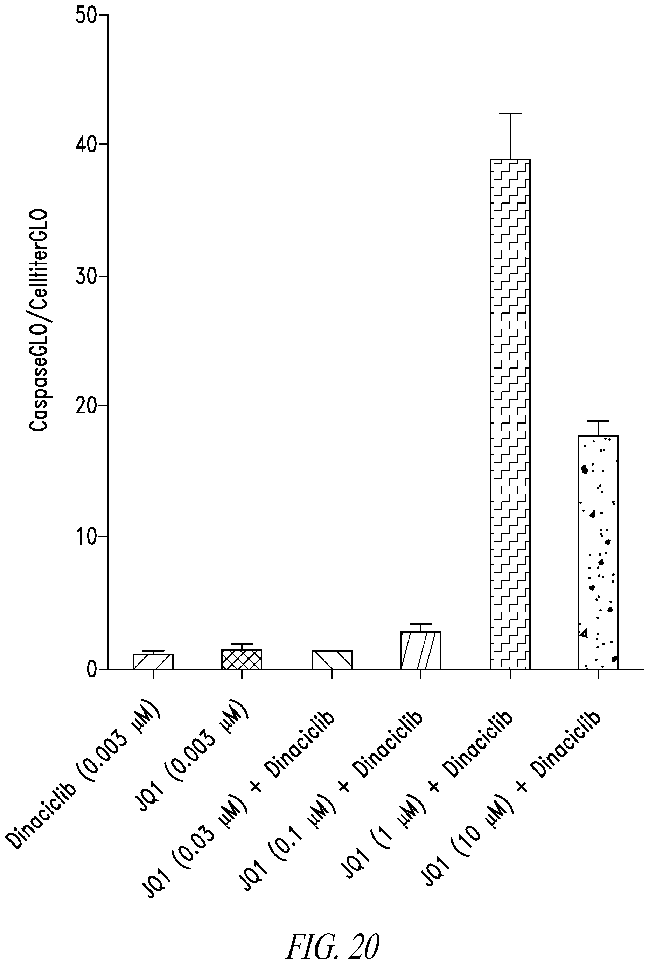

A549 cells were treated with JQ-1 at concentrations ranging from 0.03 - 10 µM for 24 hours (Figure 20). Following the treatment of JQ-1 for 24 hours, dinaciclib was added at a concentration of 3 nM. Cell Titer GLO and Caspase GLO were added and luminescence was measured at 48 hours post-dose. Escalating doses of JQ-1 combined with unchanging doses of dinaciclib demonstrated a synergistic increase in caspase activity. When a dosing of 1 µM JQ-1 is combined with a dose of 3 nM dinaciclib, a greater than 4-fold increase in apoptotic response is observed. This increase indicates dinaciclib combined with JQ-1synergistically increases apoptosis in A549 cancer cells when compared with dinaciclib or JQ-1 alone (Figure 20).

### EXAMPLE 14

### ALVOCIDIB REDUCES MCL-1 AND C-MYC EXPRESSION IN A549 CELLS

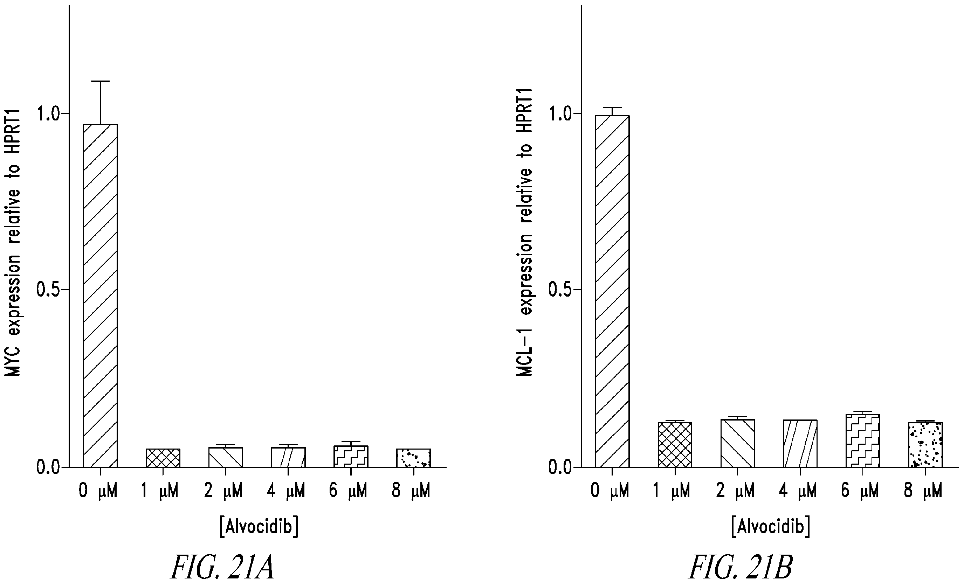

A549 cells were seeded and treated with alvocidib at concentrations ranging from 0 µM - 8 µM for 2 hours. Following the treatment of solid tumor cells, cells were harvested and mRNA was detected using RT-qPCR in the manner described in previous examples. Figure 21A shows alvocidib represses MYC mRNA expression in a dose dependent manner relative to HPRT1. Figure 21B shows alvocidib represses MCL-1 mRNA expression in a dose dependent manner relative to HPRT1.

### EXAMPLE 15

### CDK9-SPECIFIC SIRNA SYNERGIZES WITH BET INHIBITORS TO REDUCE PROTEIN EXPRESSION AND INCREASE APOPTOSIS IN A549 CELLS

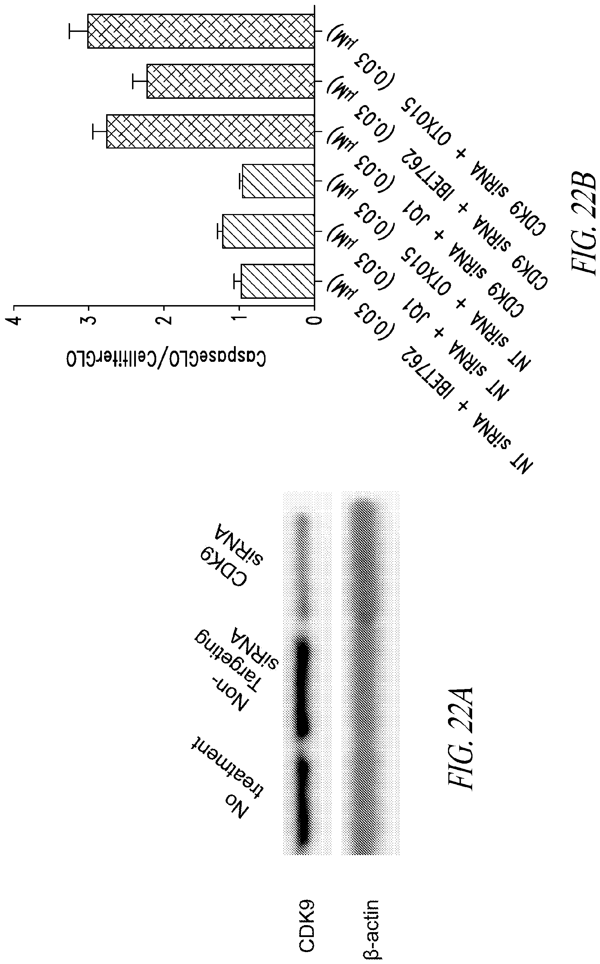

A549 cells were seeded and treated with non-targeting siRNA or CDK9-specific siRNA for 48 hours. Protein expression was measured relative to controls for untreated, non-targeting siRNA, and CDK9-specific siRNA. Figure 22A shows a definite decrease in CDK9 relative to the untreated and non-targeted siRNA while β-actin remains constant.

To assess induction of apoptosis, the above described procedure was performed, followed by treatment for 48 hours with a BET inhibitor (IBET762, JQ-1, or OTX015) at a concentration of 0.03 µM. Following 48 hour treatment with a BET inhibitor, Cell Titer GLO and Caspase GLO were added and luminescence was measured to assess cell viability and apoptosis. Figure 22B shows an approximately 2-3 fold increase in activity when cells are treated with BET inhibitors in combination with non-targeted siRNA compared to BET inhibitors with CDK9-specific siRNA.

## Claims

1. A pharmaceutical composition the composition comprising the following therapeutic agents:
i) alvocidib, or a pharmaceutically acceptable salt thereof; and
ii) a bromodomain (BRD) inhibitor, or a pharmaceutically acceptable salt thereof; for use in a method for treatment of cancer in a mammal in need thereof, wherein the therapeutic agents are co-administered, or administered sequentially.

2. The pharmaceutical composition for the use of claim 1, wherein the bromodomain inhibitor inhibits Brd4.

3. The pharmaceutical composition for the use of any one of claims 1 or 2, wherein the BRD inhibitor is BI2536, TG101209, OTX015, IBET762, IBET151, CPI-0610 or PFI-1.

4. The pharmaceutical composition for the use of claim 1, wherein the BRD inhibitor is BI2536.

5. The pharmaceutical composition for the use of claim 1, wherein the BRD inhibitor is TG101209.

6. The pharmaceutical composition for the use of claim 1, wherein the BRD inhibitor is OTX015.

7. The pharmaceutical composition for the use of claim 1, wherein the BRD inhibitor is IBET762.

8. The pharmaceutical composition for the use of claim 1, wherein the BRD inhibitor is IBET151.

9. The pharmaceutical composition for the use of claim 1, wherein the BRD inhibitor is CPI-0610.

10. The pharmaceutical composition for the use of claim 1, wherein the BRD inhibitor is PFI-1.

11. The pharmaceutical composition for the use of claims 1 to 10, wherein the BRD inhibitor is not JQ-1 and/or its analog.

12. The pharmaceutical composition for the use of any one of claims 1 to 11, wherein the cancer is a leukemia.

13. The pharmaceutical composition for the use of claim 12, wherein the leukemia is acute myeloid leukemia.

14. The pharmaceutical composition for the use of claims 1 to 11, wherein the cancer is a solid tumor.

15. The pharmaceutical composition for the use of claim 14, wherein the cancer is an adenocarcinoma of the breast, prostate or colon cancer.

## Patentansprüche

1. Pharmazeutische Zusammensetzung, wobei die Zusammensetzung die folgenden therapeutischen Mittel enthält:
i) Alvocidib oder ein pharmazeutisch akzeptables Salz davon und
ii) einen Bromodomäne (BRD)-Inhibitor oder ein pharmazeutisch akzeptables Salz davon,
zur Verwendung in einem Verfahren zur Behandlung von Krebs in einem Säuger, der diese benötigt, worin die therapeutischen Mittel gemeinsam oder aufeinanderfolgend verabreicht werden.

2. Pharmazeutische Zusammensetzung zur Verwendung gemäß Anspruch 1, worin der Bromodomäninhibitor Brd4 inhibiert.

3. Pharmazeutische Zusammensetzung zur Verwendung gemäß Anspruch 1 oder 2, worin der BRD-Inhibitor BI2536, TG101209, OTX015, IBET762, IBET151, CPI-0610 oder PFI-1 ist.

4. Pharmazeutische Zusammensetzung zur Verwendung gemäß Anspruch 1, worin der BRD-Inhibitor BI2536 ist.

5. Pharmazeutische Zusammensetzung zur Verwendung gemäß Anspruch 1, worin der BRD-Inhibitor TG101209 ist.

6. Pharmazeutische Zusammensetzung zur Verwendung gemäß Anspruch 1, worin der BRD-Inhibitor OTX015 ist.

7. Pharmazeutische Zusammensetzung zur Verwendung gemäß Anspruch 1, worin der BRD-Inhibitor IBET762 ist.

8. Pharmazeutische Zusammensetzung zur Verwendung gemäß Anspruch 1, worin der BRD-Inhibitor IBET151 ist.

9. Pharmazeutische Zusammensetzung zur Verwendung gemäß Anspruch 1, worin der BRD-Inhibitor CPI-0610 ist.

10. Pharmazeutische Zusammensetzung zur Verwendung gemäß Anspruch 1, worin der BRD-Inhibitor PFI-1 ist.

11. Pharmazeutische Zusammensetzung zur Verwendung gemäß den Ansprüchen 1 bis 10, worin der BRD-Inhibitor nicht JQ-1 und/oder dessen Analog ist.

12. Pharmazeutische Zusammensetzung zur Verwendung gemäß den Ansprüchen 1 bis 11, worin der Krebs eine Leukämie ist.

13. Pharmazeutische Zusammensetzung zur Verwendung gemäß Anspruch 12, worin die Leukämie akute myeloische Leukämie ist.

14. Pharmazeutische Zusammensetzung zur Verwendung gemäß den Ansprüchen 1 bis 11, worin der Krebs ein fester Tumor ist.

15. Pharmazeutische Zusammensetzung zur Verwendung gemäß Anspruch 14, worin der Krebs ein Adenokarzinom der Brust, Prostata oder Kolonkrebs ist.

## Revendications

1. Composition pharmaceutique, la composition comprenant les agents thérapeutiques suivants :
i) l'alvocidib, ou un sel pharmaceutiquement acceptable de celui-ci ; et
ii) un inhibiteur de bromodomaine (BRD), ou un sel pharmaceutiquement acceptable de celui-ci ;
pour une utilisation dans une méthode de traitement d'un cancer chez un mammifère en ayant besoin,
dans laquelle les agents thérapeutiques sont co-administrés, ou administrés séquentiellement.

2. Composition pharmaceutique pour l'utilisation selon la revendication 1, dans laquelle l'inhibiteur de bromodomaine inhibe Brd4.

3. Composition pharmaceutique pour l'utilisation selon l'une quelconque des revendications 1 ou 2, dans laquelle l'inhibiteur de BRD est BI2536, TG101209, OTX015, IBET762, IBET151, CPI-0610 ou PFI-1.

4. Composition pharmaceutique pour l'utilisation selon la revendication 1, dans laquelle l'inhibiteur de BRD est BI2536.

5. Composition pharmaceutique pour l'utilisation selon la revendication 1, dans laquelle l'inhibiteur de BRD est TG101209.

6. Composition pharmaceutique pour l'utilisation selon la revendication 1, dans laquelle l'inhibiteur de BRD est OTX015.

7. Composition pharmaceutique pour l'utilisation selon la revendication 1, dans laquelle l'inhibiteur de BRD est IBET762.

8. Composition pharmaceutique pour l'utilisation selon la revendication 1, dans laquelle l'inhibiteur de BRD est IBET151.

9. Composition pharmaceutique pour l'utilisation selon la revendication 1, dans laquelle l'inhibiteur de BRD est CPI-0610.

10. Composition pharmaceutique pour l'utilisation selon la revendication 1, dans laquelle l'inhibiteur de BRD est PFI-1.

11. Composition pharmaceutique pour l'utilisation selon les revendications 1 à 10, dans laquelle l'inhibiteur de BRD n'est pas JQ-1 et/ou son analogue.

12. Composition pharmaceutique pour l'utilisation selon l'une quelconque des revendications 1 à 11, dans laquelle le cancer est une leucémie.

13. Composition pharmaceutique pour l'utilisation selon la revendication 12, dans laquelle la leucémie est une leucémie myéloïde aiguë.

14. Composition pharmaceutique pour l'utilisation selon les revendications 1 à 11, dans laquelle le cancer est une tumeur solide.

15. Composition pharmaceutique pour l'utilisation selon la revendication 14, dans laquelle le cancer est un adénocarcinome du cancer du sein, de la prostate ou du côlon.
